# EUROPÄISCHE PATENTSCHRIFT

(11) **EP 1 913 890 B1**
(45) Veröffentlichungstag und Bekanntmachung des Hinweises auf die Patenterteilung: **15.04.2009**
(21) Anmeldenummer: 06122639.5
(22) Anmeldetag: 20.10.2006
(51) Int. Cl.: A61B 19/00

(54) **Markernavigationsvorrichtung insbesondere für medizinische Zwecke**
Marker-navigation device in particular for medical use
Dispositif de navigation avec un marqueur en particulier pour usage medicaux

(43) Veröffentlichungstag der Anmeldung: 23.04.2008
(73) Patentinhaber: BrainLAB AG, 85622 Feldkirchen (DE)
(72) Erfinder: Immerz, Martin, 82166, Gräfelfing (DE); Tuma, Gregor, 81675, München (DE)
(74) Vertreter: Schwabe - Sandmair - Marx

(56) Entgegenhaltungen:
- EP-A- 1 645 228
- US-A1- 2006 142 657
- US-B1- 6 466 815

## Beschreibung

Die vorliegende Anmeldung betrifft eine Markernavigationsvorrichtung zur Detektion einer Markereinrichtung insbesondere für medizinische Zwecke, insbesondere zur Navigation, d. h. Bestimmung der Lage beweglicher Gegenstände, an denen die Markereinrichtung angebracht ist, insbesondere in Operationsräumen.

Gegenwärtig werden mittels einer Detektionseinrichtung (z. B. Kamera oder Ultraschalldetektor) Markereinrichtungen detektiert. Bei den Markereinrichtungen handelt es sich typischerweise um drei Marker, die in fester und vorbestimmter relativer Lage zueinander angeordnet sind und insbesondere mechanisch verbunden sind. Die Marker können passive oder aktive Marker sein, wobei die passiven Marker Wellen und/oder Strahlung reflektieren, die in ihre Richtung ausgesendet wird und die aktiven Marker selbst Ursprung der Strahlung und/oder Wellen sind. Die von den (aktiven oder passiven) Markern ausgehenden Signale, bei denen es sich um Wellensignale oder Strahlungssignale handeln kann, werden von einer Detektionsvorrichtung (z. B. Kamera) detektiert. Um eine Ausgangsposition der Markereinrichtung relativ zu der Detektionseinrichtung festzulegen, wird dabei vorzugsweise die Markereinrichtung bewegt, um der Detektionseinrichtung verschiedene Ansichten der Markereinrichtung zu bieten. Auf dieser Grundlage kann dann in bekannter Weise die relative Lage der Markereinrichtung relativ zu der Detektionseinrichtung, insbesondere in einem im Raum ruhenden Bezugssystem bestimmt werden. In diesem Zusammenhang wird auf die DE 196 39 615 A1 und die entsprechende US-Veröffentlichung 6,351,659 hingewiesen, die hiermit durch Bezugnahme in die vorliegende Offenbarung aufgenommen werden.

Eine Markereinrichtung hat typischerweise drei Marker. Es können aber auch zwei Marker sein oder mehr als drei Marker. Dabei kann es vorkommen, dass einer der Marker einen anderen Marker so aus der Sicht der Detektionseinrichtung verdeckt, dass der Marker von der Detektionseinrichtung nicht mehr vollständig oder nur teilweise detektierbar ist, insbesondere, betrachtet von der Detektionseinrichtung, im optischen Schatten des einen Markers liegt oder von diesem optischen Schatten zum Teil erfasst wird.

Typischerweise hat eine Markereinrichtung drei Marker. In dieser Situation kann ein Verdecken eines Markers durch einen anderen Marker nur dann auftreten, wenn alle drei Marker sich in einer Ebene befinden.

Wird einer der Marker durch einen anderen Marker verdeckt, so leidet die Genauigkeit der Detektion der Lage der Markereinrichtung erheblich und kann unzuverlässig werden. Dies ist insbesondere im medizinischen Bereich riskant, sodass bei einer derartigen Verdeckungssituation der Bediener darauf aufmerksam gemacht werden sollte und/oder eine Anzeige der aktuellen Lage der Markereinrichtung unterbleiben sollte.

Falls nur drei Marker vorgesehen sind, so spannen diese eine Ebene auf. Innerhalb eines vorbestimmten Raumwinkels, durch den mittig ein normal auf der Ebene stehender Vektor verläuft, ist demnach eine Detektion aller drei Marker möglich, falls sich die Detektionseinrichtung in diesem Raumwinkel befindet. Eine Steuerung könnte auf eine derartige Bedingung zurückgreifen, um ein Warnsignal abzugeben, falls sich die Detektionseinrichtung nicht innerhalb dieses Raumwinkels befindet oder diesen verlässt. Eine derartige Begrenzung der Betriebsfähigkeit der erfindungsgemäßen Markernavigationsvorrichtung nutzt jedoch nicht alle möglichen Positionen der Detektion aller Marker. Auch wenn sich die Detektionseinrichtung in derselben Ebene befindet wie die drei Marker, also bei einem Raumwinkel von 90°, gibt es noch relative Lagen zwischen der Markereinrichtung und der Detektionseinrichtung, bei denen alle drei Marker für die Detektionseinrichtung detektierbar sind.

Die Detektion von Markereinrichtungen ist aus EP 1 645 228 A1 bekannt.

Aufgabe der Erfindung ist, eine Markernavigationsvorrichtung zu schaffen, die bei möglichst vielen relativen Lagen zwischen Markereinrichtung und Detektionseinrichtung eine Bestimmung der Lage der Markereinrichtung ermöglicht, ohne die Verlässlichkeit der Bestimmung der Lage der Markereinrichtung zu gefährden.

Vorstehende Aufgabe wird durch die Gegenstände des unabhängigen Anspruches gelöst. Vorteilhafte Ausführungsformen gehen aus den abhängigen Ansprüchen hervor.

Die erfindungsgemäße Markernavigationsvorrichtung erlaubt bevorzugt die Bestimmung und/oder die Verfolgung der Lage einer Markereinrichtung. Dabei ist die Markereinrichtung vorzugsweise an einem Gegenstand, insbesondere einer Körperstruktur (z. B. Knochen oder Knorpel) oder einem (medizinischen) Instrument angebracht. Somit erlaubt die Bestimmung der Lage der Markereinrichtung die Bestimmung der Lage des Gegenstandes. Insbesondere erfolgt die Bestimmung der Lage fortlaufend oder wiederholt, sodass eine Verfolgung (Tracking) der Lage der Markereinrichtung und somit des Gegenstandes durchgeführt werden kann.

Die Lage der Markereinrichtung wird bevorzugt durch die Position der Markereinrichtung in einem vorbestimmen Bezugssystem bestimmt. Vorzugsweise wird als Bezugssystem ein Bezugssystem verwendet, in dem die Detektionseinrichtung ruht. Insbesondere wird die Lage der Markereinrichtung durch die Positionen der Marker, insbesondere der Mittelpunkte der Marker in einem Bezugssystem bestimmt. Die Positionen können zum Beispiel mit kartesischem Koordinaten oder Kugelkoordinaten beschrieben werden. Die relative Lage von einem Teil (z. B. Detektionseinrichtung oder Markereinrichtung) zu einem anderen Teil (z. B. Markereinrichtung) kann insbesondere durch Raumwinkel und/oder Abstände und/oder Koordinaten (in einem Bezugssystem) und/oder Vektoren beschrieben werden und wird vorzugsweise aus den die Lage beschreibenden Positionen z. B. mittels eines Programms errechnet, das auf einem Computer läuft.

Der hierin verwendete Begriff "relative Lage" oder der Ausdruck "Lage eines Teils A relativ zu einem Teil B" umfasst also den Begriff der relativen Positionen zwischen den zwei Teilen, insbesondere zwischen den Markern oder zwischen einem Marker und der Detektionseinrichtung. Insbesondere werden Schwerpunkte oder Mittelpunkte der Teile als punktförmiger Bezugspunkt zum Festlegen einer Position gewählt. Ist die Position eines Teils in einem Bezugssystem bekannt, so kann basierend auf der relativen Lage zweier Teile aus der Position eines der zwei Teile die Position des anderen der zwei Teile berechnet werden.

Insbesondere falls die Markereinrichtung nur zwei Marker umfasst, ist bevorzugt eine Startposition bekannt, und die Markernavigationsvorrichtung erlaubt dann die Verfolgung der Lage der Markereinrichtung bei Bewegung der Markereinrichtung im Raum.

Die Markereinrichtung gemäß der vorliegenden Erfindung umfasst also vorzugsweise mindestens zwei Marker, insbesondere drei Marker. Die Abmessungen der Marker und die relative Lage der Marker zueinander ist bekannt und liegt insbesondere als vorbekannte Daten einer Datenverarbeitungseinrichtung vor. Vorzugsweise ist auch die Form der Marker bekannt.

Weiter umfasst die erfindungsgemäße Markernavigationsvorrichtung vorzugsweise eine Detektionseinrichtung, die Signale von den mindestens zwei Markern detektiert. Wie zuvor ausgeführt handelt es sich hierbei um von den Markern ausgehende Signale, die entweder aktiv von den Markern ausgesendet werden oder von den Markern reflektiert werden. Im letzteren Fall ist vorzugsweise weiter eine Signalsendequelle beispielsweise eine Infrarotlichtquelle vorgesehen, die Signale (z. B. Ultraschallpulse oder Infrarotlicht) in Richtung auf die passiven Marker aussendet, wobei die passiven Marker die Signale reflektieren. Eine Datenverarbeitungseinrichtung, insbesondere ein Computer, erlaubt die Berechnung der relativen Lage der Markereinrichtung relativ zur Detektionseinrichtung, insbesondere die Berechnung der Lage der Markereinrichtung in einem Bezugssystem, in dem die Detektionseinrichtung ruht, also beispielsweise in einem Bezugssystem, das in einem Operationssaal ruht.

Wie bereits oben ausgeführt, gibt es relative Lagen zwischen den Markern der Markereinrichtung und der Detektionseinrichtung, bei denen nicht alle Marker detektierbar sind (d. h. eine Detektion unmöglich ist) und/oder insbesondere die von den Markern ausgehenden Signale nicht klar voneinander unterscheidbar sind, also eine Detektion beeinträchtigt ist. Derartige relative Lagen werden hierin als erste relative Lagen bezeichnet. Eine Beeinträchtigung der Detektion bedeutet hierbei insbesondere, dass sich die von beiden Markern ausgehenden Signale zumindest teilweise auf einem oder mehreren signalempfindlichen Elementen der Detektionseinrichtung überlappen bzw. gleichzeitig detektiert werden. Im optischen Fall also wird die elektromagnetische Strahlung (Licht oder infrarotes Licht), die von beiden Markern ausgeht, zumindest teilweise von einem oder mehreren fotoempfindlichen Elementen gleichzeitig gemessen. Beeinträchtigt bedeutet insbesondere auch, dass die von einem Marker ausgehenden Signale durch einen anderen Marker abgeschwächt werden. Dies ist insbesondere dann der Fall, wenn die von dem einen Marker ausgehende Strahlung oder die ausgehende Wellen von dem anderen Marker zumindest teilweise blockiert werden. Der eine Marker befindet sich also aus Sicht der Detektionseinrichtung zumindest teilweise im Schatten des anderen Markers. Abhängig von der angewandten Technik der Datenverarbeitung (z. B. Mustererkennung) kann aus der Sicht der Detektionseinrichtung ein überlappendes Signal noch ausreichend sein, um die Signale zweier Marker im ausreichenden Maße trennen zu können. Es kann aber auch ein deutlicher Abstand zwischen den Markern erforderlich sein, sodass beispielsweise Detektionselemente, die keine Signale empfangen, zwischen ersten Detektionselementen, die Signale von einem ersten Marker empfangen, und zweiten Detektionselementen, die Signale von einem zweiten Marker empfangen, liegen.

Auch können bei einer Ausführungsform der Erfindung unter den ersten relativen Lagen noch solche verstanden werden, bei denen zwar eine Signaltrennung noch möglich ist, aber bei einer weiteren Verlagerung der Markereinrichtung in einem vorbestimmten Umfang und in einer vorbestimmten Art und Weise dies nicht mehr möglich wäre. Auch dies wird bei einer Ausführungsform der Erfindung als Beeinträchtigung verstanden, da eine sichere Bestimmung der Lage der Markereinrichtung oder eine Verfolgung der Lage gefährdet ist. Die ersten relativen Lagen umfassen deshalb vorzugsweise einen "Sicherheitskorridor" (beispielsweise ein Raum zwischen einem zweiten und ersten Mantel), um so rechtzeitig einen Bediener auf eine mögliche Unterbrechung der Anzeige einer Lage der Markereinrichtung hinzuweisen.

Übrige relative Lagen zwischen den Markern der Markereinrichtung und der Detektionseinrichtung, die insbesondere keine ersten relativen Lagen sind und/oder eine Detektion sämtlicher Marker erlauben, werden als zweite relative Lagen bezeichnet.

In dem vorgenannten "Sicherheitskorridor" ist zwar noch eine getrennte Detektion der Marker möglich, bei einer weiteren Verlagerung der Markereinrichtung in einem bestimmten Umfang (und in eine bestimmte Richtung) wäre dies aber nicht mehr möglich.

Mittels der Datenverarbeitungseinrichtung werden bevorzugt die relativen Lagen zwischen der Markereinrichtung und der Detektionseinrichtung berechnet. Die Berechnung erfolgt vorzugsweise basierend auf den bekannten Abmessungen der Marker der Markereinrichtung und den bekannten relativen Lagen zwischen den einzelnen Markern der Markereinrichtung (also beispielsweise basierend auf den Abständen der Marker und den Winkeln des Dreiecks, dessen Eckpunkte die Mittelpunkte der Marker sind). Da vorzugsweise die Detektionseinrichtung zwei räumlich getrennte Detektoren (z. B. zwei Kameras) umfasst, ist ein räumliches Sehen möglich, und aufgrund Detektionssignale, genauer aufgrund der detektierten Abstände zwischen den Markern und den detektierten Winkeln des vorgenannten Dreiecks lässt sich die aktuelle relative Lage zwischen der Markereinrichtung und der Detektionseinrichtung bestimmen.

Sind nun die ersten und zweiten relativen Lagen sowie die aktuelle relative Lage bekannt, so kann die aktuelle relative Lage mit der ersten und/oder zweiten relativen Lage verglichen werden, um zu bestimmen, ob die aktuelle relative Lage eine Lage der zweiten relativen Lagen ist und/oder der ersten relativen Lagen ist. Diese Bestimmung wird vorzugsweise mittels der Datenverarbeitungseinrichtung durchgeführt. Die ersten und/oder zweiten relativen Lagen werden vorzugsweise durch geometrische Größen (z. B. Winkel) und/oder Figuren (z. B. Konus, Zylinder) definiert, wie weiter unten näher ausgeführt wird. Insbesondere werden die ersten relativen Lagen durch geometrische Figuren (z. B. Konus) in einem Bezugssystem beschrieben, in dem die Markereinrichtung ruht.

Vorzugsweise umfasst die Markernavigationsvorrichtung ein Warnmittel, das insbesondere so ausgebildet ist, dass es eine Meldung ausgibt. Diese Meldung ist oder umfasst die Ausgabe einer Mitteilung, beispielsweise ein Alarmsignal, falls die aktuelle relative Lage eine erste relative Lage ist, also falls beispielsweise einer der Marker durch den anderen abgedeckt wird. Zusätzlich oder alternativ wird die Ausgabe der Meldung so ausgestaltet, dass eine Mitteilung der aktuellen relativen Lage, also beispielsweise eine Anzeige der aktuellen relativen Lage (auf einem Monitor) unterbunden wird. Beispielsweise zeigt der Monitor dann nur einen schwarzen Schirm oder einen Warnhinweis.

Im Folgenden werden beispielhaft Eigenschaften der ersten relativen Lagen beschrieben. Die ersten relativen Lagen liegen vorzugsweise auf Geraden, die durch mindestens zwei der Marker hindurch gehen. Insbesondere liegen sie innerhalb zweier Kegelspitzen, deren Mantel durch Geraden gebildet werden, die den Rand des kreisförmigen Querschnitts zweier Marker berühren und sich an einem Punkt schneiden, der auf einer die Mittelpunkte der beiden Kugeln verbindenden Linie liegt. Insbesondere liegt der Schnittpunkt der Geraden (d. h. der Kegelspitzen) in der Mitte zwischen den beiden Kugeln, falls diese gleichgroß ausgebildet sind.

Vorzugsweise liegen die Marker insbesondere auch auf Geraden, die durch zumindest einen der Marker hindurchgehen und an einem anderen Marker in einem vorbestimmten Abstand vorbeilaufen. Falls die Geraden in einem bestimmten Abstand an den Markern vorbeilaufen ergibt sich ein gewisser Sicherheitsabstand ("Sicherheitskorridor"), der es erlaubt frühzeitig zu erkennen, dass bei einer weiteren Bewegung der Markereinrichtung, so dass einer der Marker in den Schatten des anderen läuft, möglicherweise einer der Marker verdeckt wird.

Ein Sicherheitskorridor ergibt sich, falls die ersten relativen Lagen auf Geraden liegen, die in einem vorbestimmten Abstand oder in einem Abstand, der weniger als den vorbestimmten Abstand ist, an den Markern vorbeilaufen. Der vorbestimmte Abstand liegt vorzugsweise in einem Bereich bis zu dem dreifachen des Durchmessers des Markers, besonders bevorzugt in einem Bereich bis zu dem einfachen oder halbem oder viertel des Markerdurchmessers, insbesondere in einem Bereich >10%, insbesondere >50% des Markerdurchmessers.

Die ersten relativen Lagen lassen sich auch dadurch beschreiben, dass sie innerhalb eines gedachten Mantels liegen. Vorzugsweise ist der Mantel dabei so gestaltet, dass er die Marker umgibt, wobei der Mantel vorzugsweise mindestens einen der zwei Marker berührt. Alternativ kann der Mantel auch in einem vorbestimmten Abstand von mindestens einem der zwei Marker verlaufen. Beispiele für einen vorbestimmten Abstand wurden bereits oben genannt. Ein Beispiel für einen vorgenannten Sicherheitskorridor kann so beschrieben werden, dass ein Mantel (Kernmantel) einen Kernbereich umgibt, wenn er die zwei Marker berührt. Der Zwischenraum zwischen diesem Kernmantel und einem Mantel, der in einem vorbestimmten Abstand zu den Markern verläuft, kann als Sicherheitskorridor genutzt werden. Tritt aufgrund der Bewegung der Markereinrichtung die aktuelle relative Lage der Detektionseinrichtung relativ zur Markereinrichtung in den Sicherheitskorridor ein, so kann ein Warnsignal abgegeben werden. Wird die Bewegung weiterverfolgt und die relative Lage tritt in den Bereich des Kernmantels ein, so kann dann die Anzeige der aktuellen relativen Lage unterbunden werden und/oder ein anderes, insbesondere intensiveres Warnsignal abgegeben werden. Der vorgenannte Mantel kann bei kugelförmigen Markern insbesondere eine zylindrische Gestalt einnehmen, insbesondere falls es sich um "einäugige" Detektoren handelt. Wie bereits oben ausgeführt, sind jedoch vorzugsweise (mindestens) zwei Detektoren in der Detektionseinrichtung vorhanden. Insbesondere in diesem Fall wird eine Mantelfläche bevorzugt, die sich zwischen den Markern verjüngt und die sich insbesondere durch zwei an ihrer Spitze berührende Konusse (Kegel) mit ineinander übergehender Konusachse beschreiben lässt.

Vorzugsweise ist die Markereinrichtung so ausgebildet, dass eine Anzeige der aktuellen (basierend auf den aktuellen Detektionssignalen berechneten) relativen Lage selbst dann erfolgt, wenn sich die Detektionseinrichtung und die Markereinrichtung in derselben Ebene befinden, aber die Detektionseinrichtung nicht eine erste relative Lage einnimmt. Anders ausgedrückt gibt es vorzugsweise zweite relative Lagen, die in derselben Ebene liegen, die durch die Marker festgelegt wird.

Im Folgenden wird eine Ausführungsform detailliert beschrieben. Dabei werden weitere Merkmale der Erfindung offenbart.

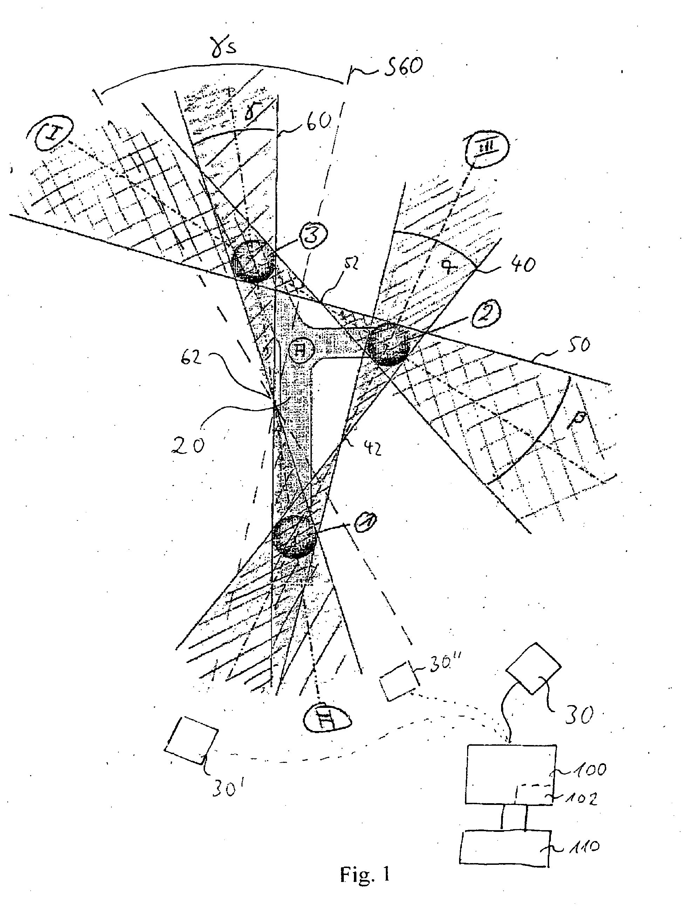

Die Fig. 1 zeigt eine erfindungsgemäße Markernavigationsvorrichtung.

In Fig. 1 ist eine erfindungsgemäße Markernavigationsvorrichtung zu sehen. Diese umfasst einen Referenzstern 20 mit Markerkugeln 1, 2 und 3, die durch ein Gestänge A verbunden sind. Der Referenzstern 20 stellt ein Beispiel für eine Markereinrichtung dar. Die Markerkugeln, bei denen es sich insbesondere um passive Markerkugeln handelt, die selbst keine Strahlung aussenden aber Strahlung oder Wellen reflektieren, werden durch eine Detektionseinrichtung 30 detektiert. Für die Detektionseinrichtung 30 sind in der Fig. 1 zwei beispielhafte Stellungen gezeichnet. Die erste Stellung ist mit dem Bezugszeichen 30 versehen, die zweite Stellung mit dem Bezugszeichen 30'.

Konusförmige Mäntel mit dem Bezugszeichen 40, 50 und 60 umgeben die Markerkugeln 1, 2 und 3. Der konusförmige Mantel 60 betrifft die Markerkugeln 1 und 3,. der konusförmige Mantel 40 betrifft die Markerkugeln 2 und 1 und der konusförmige Mantel 50 betrifft die Markerkugeln 2 und 3. Die konusförmigen Mäntel werden durch Geraden gebildet bzw. aufgespannt, die die Außenseite der Markerkugeln berühren und die sich in der Mitte zwischen zwei Markerkugeln auf einer Achse, die den Mittelpunkt der Markerkugeln durchläuft, schneiden. Die die Mittelpunkte der Markerkugeln durchlaufenden Achsen stellen auch die zentralen Achsen der konusförmigen Mäntel dar, die rotationssymmetrisch um diese Achsen ausgebildet sind. Die Achsen sind mit I, II und III bezeichnet. Befindet sich die Detektionseinrichtung an der mit 30 bezeichneten Position, so verdeckt keine der Markerkugeln die andere. Befindet sie sich aber an der an mit 30' bezeichneten Stellung, so verdeckt die Markerkugel 1 die Markerkugel 2. Anders ausgedrückt die Markerkugel 2 befindet sich im Schatten der Markerkugel 1. In diesem Fall ist eine Detektion sämtlicher Markerkugeln nicht mehr möglich und die Anzeige der aktuellen relativen Lage der Markereinrichtung erfolgt vorzugsweise nicht. Die aktuelle relative Lage der Detektionseinrichtung 30 wird mit einer Datenverarbeitungseinrichtung 100 berechnet. Die Datenverarbeitungseinrichtung 100 ist vorzugsweise elektrisch bzw. signaltechnisch mit der Detektionseinrichtung 30 (oder 30', siehe gestrichelte Linie) verbunden. Die Datenverarbeitungseinrichtung 100 gibt die berechneten Daten zu einer Anzeigeeinrichtung 110 aus, die die aktuelle relative Lage anzeigt, falls dies nicht durch das Warnmittel 102, das durch ein Softwareprogramm ausgebildet sein kann und auf der Datenverarbeitungseinrichtung 100 laufen kann, verhindert wird. Alternativ oder zusätzlich zur Anzeigeeinrichtung 110 kann die Mitteilung der aktuellen Lage beispielsweise auch akustisch erfolgen.

Die mit den Mantelflächen 40, 50 und 60 ummantelten Konusse schließen jeweilig Raumwinkel α, β und γ ein. Wie aus Fig. 1 ersichtlich, sind die Raumwinkel α, β und γ unterschiedlich. Dies liegt an den unterschiedlichen Abständen zwischen den Markerkugeln 1 und 3, 3 und 2, und 2 und 1.

Mit S60 ist in Fig. 1 ein Mantel bezeichnet, der den Mantel 60 (rotationssymmetrisch) umgibt. Der Mantel S60 spannt einen Raumwinkel γₛ auf. Der Mantel S60 umschreibt einen Sicherheitskorridor, der sich zwischen dem Mantel 60 und dem Mantel S60 befindet. In der bei 30" bezeichneten Stellung der Detektionseinrichtung ist eine Detektion aller drei Markerkugeln möglich. Jedoch ist eine Detektion gefährdet, falls sich die Markereinrichtung weiter so bewegt, dass die Detektionseinrichtung 30 in den Mantel 60 gelangt. In dieser Stellung kann ein Warnsignal ausgegeben werden, das darauf hinweist, dass möglicherweise bei einer Weiterverfolgung der Bewegung eine Anzeige der Lagen nicht mehr möglich ist. Der in Fig. 1 gezeigte Sicherheitskorridor liegt außerhalb des Raumwinkels γ aber innerhalb des Raumwinkels γₛ. Der zuvor genannte Mantel S60 wird hierin auch als erster Mantel bezeichnet und die zuvor genannten Mäntel 40, 50 und 60 werden als zweite Mäntel bezeichnet. Der vorgenannte "Sicherheitskorridor" ist also der Raum zwischen dem ersten Mantel S60 und dem zweiten Mantel 60. Die ersten Mäntel werden vorzugsweise so gestaltet, dass in einer Ebene, die durch die Markerkugeln aufgespannt wird, zweite relative Lagen vorhanden sind, die außerhalb der ersten Mäntel liegen, aber sich in dieser Ebene befinden.

In Fig. 1 sind die zweiten relativen Lagen diejenigen, die sich außerhalb der Mantelflächen 40, 50 und S60 befinden.

## Patentansprüche

1. Markernavigationsvorrichtung zur Bestimmung und/oder Verfolgung der Lage einer Markereinrichtung insbesondere für medizinische Zwecke, insbesondere zur Navigation;
mit einer Markereinrichtung (20), die mindestens zwei Marker (1, 2, 3) mit bekannten Abmessungen und in bekannter relativer Lage zueinander aufweist;
mit einer Detektionseinrichtung (30, 30', 30"), die Signale von den mindestens zwei Markern (1, 2, 3) detektiert;
wobei die Detektionseinrichtung (30, 30', 30") so ausgebildet ist, dass
in ersten relativen Lagen (40, 50, 60, S60) der Detektionseinrichtung (30', 30") relativ zur Markereinrichtung (20) eine Detektion eines der Marker (2) mittels der Detektionseinrichtung (30', 30") auf Grund der Lage eines anderen der Marker (1) beeinträchtigt oder unmöglich ist, und
in übrigen, zweiten relativen Lagen relativ zu der Markereinrichtung (20) sämtliche Marker (1, 2, 3) der Markereinrichtung (20) mittels der Detektionseinrichtung zu detektieren sind;
mit einer Datenverarbeitungseinrichtung (100), **dadurch gekennzeichnet, dass** die Datenverarbeitungseinrichtung ausgebildet ist, dass sie
die ersten relativen Lagen (40, 50, 60, S60) basierend auf den relativen Lagen zwischen den einzelnen Markern (1, 2, 3) der Markereinrichtung (20) und basierend auf den Abmessungen der Marker (1, 2, 3) der Markereinrichtung (20) berechnet; und
basierend auf den detektierten Signalen, den bekannten relativen Lagen der Marker (1, 2, 3) zueinander und den bekannten Abmessungen der Marker (1, 2, 3) die aktuelle relative Lage zwischen der Markereinrichtung (20) und der Detektionseinrichtung (30, 30', 30") berechnet und bestimmt, ob die aktuelle relative Lage eine Lage der ersten relativen Lagen (40, 50, 60, S60) ist; und dass sie
mit einem Warnmittel (102) versehen ist, das, wenn die aktuelle relative Lage eine der ersten relativen Lagen (40, 50, 60, S60) ist, eine Meldung ausgibt.

2. Markerdetektionsvorrichtung nach Anspruch 1, wobei die ersten relativen Lagen (40, 50, 60, S60) mindestens eine der folgenden Bedingungen erfüllen:
a) sie liegen auf Geraden, die durch mindestens zwei der Marker hindurchgehen;
b) sie liegen auf Geraden, die durch einen der Marker hindurchgehen und durch einen anderen der Marker in einem vorbestimmten Abstand oder weniger als dem vorbestimmten Abstand vorbeilaufen; und/oder
c) sie liegen auf Geraden, die an zwei der Marker in einem vorbestimmten Abstand oder weniger als dem vorbestimmten Abstand vorbeilaufen.

3. Markerdetektionsvorrichtung nach Anspruch 1 oder 2, wobei die ersten relativen Lagen (40, 50, 60, S60) auf Geraden liegen, die sich zwischen zwei Markern schneiden.

4. Markerdetektionsvorrichtung nach einem der Ansprüche 1 bis 3, wobei die ersten relativen Lagen innerhalb eines gedachten Mantels (40, 50, 60, S60) liegen, wobei der Mantel (40, 50, 60, S60) zwei der Marker (1, 2, 3) umgibt und mindestens einen der zwei Marker (1, 2, 3) berührt oder in einem vorbestimmten Abstand von mindestens einem der zwei Marker (1, 2, 3) verläuft.

5. Markerdetektionsvorrichtung nach Anspruch 4, wobei der Mantel zwischen den zwei Markern eine engste Querschnittsfläche oder einen Querschnittspunkt (42, 52, 62) aufweist und sich ausgehend hiervon stetig erweitert.

6. Markerdetektionsvorrichtung nach Anspruch 4 oder 5, wobei die Marker einen runden Querschnitt aufweisen und der Mantel konusförmig ist.

7. Markerdetektionsvorrichtung nach einem der Ansprüche 1 bis 6, bei welcher das Warnmittel (102) eine erste Meldung ausgibt, wenn die aktuelle Lage eine der ersten relativen Lagen ist und innerhalb eines ersten gedachten Mantels (S60) aber nicht innerhalb eines zweiten gedachten Mantels (60) liegt, und eine zweite Meldung ausgibt, wenn die aktuelle Lage innerhalb des zweiten Mantels (60) liegt, wobei der erste Mantel (S60) den zweiten Mantel (60) umgibt, zumindest der erste und zweite Mantel zwei Marker umgibt die erste Meldung sich von der zweiten Meldung unterscheidet und der erste Mantel (S60) von den Markern (1, 2, 3) beabstandet ist.

8. Markerdetektionsvorrichtung nach einem der Ansprüche 1 bis 7, mit einem Anzeigemittel (110) zur Anzeige einer der Markereinrichtung entsprechenden Lage basierend auf der berechneten aktuellen relativen Lage, selbst wenn die Marker (1, 2, 3) sich in einer Ebene befinden und falls die aktuelle relative Lage eine der zweiten relativen Lagen ist.

9. Markerdetektionsvorrichtung nach einem der vorhergehenden Ansprüche, wobei das Ausgeben der Meldung durch das Warnmittel (102) die Mitteilung eines Alarmsignals und/oder das Unterbinden der Mitteilung der aktuellen relativen Lage umfasst.

## Claims

1. A marker navigation device for determining and/or tracking the location of a marker means, in particular for medical purposes, in particular for navigation;
comprising a marker means (20) which comprises at least two markers (1, 2, 3) which have known dimensions and are in a known location relative to each other;
comprising a detection means (30, 30', 30") which detects signals from the at least two markers (1, 2, 3);
wherein the detection means (30, 30', 30") is formed such that:
in first relative locations (40, 50, 60, S60) of the detection means (30', 30") relative to the marker means (20), detecting one of the markers (2) by means of the detection means (30', 30") is restricted or impossible due to the location of one of the other markers (1); and
in remaining, second relative locations relative to the marker means (20), all the markers (1, 2, 3) of the marker means (20) can be detected by means of the detection means;
comprising a data processing means (100), **characterised in that** the data processing means is formed such that it:
calculates the first relative locations (40, 50, 60, S60) on the basis of the relative locations between the individual markers (1, 2, 3) of the marker means (20) and on the basis of the dimensions of the markers (1, 2, 3) of the marker means (20); and
calculates the current relative location between the marker means (20) and the detection means (30, 30', 30") on the basis of the detected signals, the known locations of the markers (1, 2, 3) relative to each other and the known dimensions of the markers (1, 2, 3), and determines whether the current relative location is one of the first relative locations (40, 50, 60, S60); and **in that** it
is provided with a warning apparatus (102) which outputs a message if the current relative location is one of the first relative locations (40, 50, 60, S60).

2. The marker detection device according to claim 1, wherein the first relative locations (40, 50, 60, S60) meet at least one of the following conditions:
a) they lie on straight lines which pass through at least two of the markers;
b) they lie on straight lines which pass through one of the markers and pass by one of the other markers at a predetermined distance or less than the predetermined distance; and/or
c) they lie on straight lines which pass by two of the markers at a predetermined distance or less than the predetermined distance.

3. The marker detection device according to claim 1 or 2, wherein the first relative locations (40, 50, 60, S60) lie on straight lines which intersect between two markers.

4. The marker detection device according to any one of claims 1 to 3, wherein the first relative locations lie within an imaginary boundary surface (40, 50, 60, S60), wherein the boundary surface (40, 50, 60, S60) surrounds two of the markers (1, 2, 3) and touches at least one of the two markers (1, 2, 3) or passes at a predetermined distance from at least one of the two markers (1, 2, 3).

5. The marker detection device according to claim 4, wherein the boundary surface exhibits its narrowest cross-sectional area or a cross-sectional point (42, 52, 62), from which it continuously widens, between the two markers.

6. The marker detection device according to claim 4 or 5, wherein the markers exhibit a round cross-section and the boundary surface is cone-shaped.

7. The marker detection device according to any one of claims 1 to 6, wherein the warning apparatus (102) outputs a first message if the current location is one of the first relative locations and lies within a first imaginary boundary surface (S60) but not within a second imaginary boundary surface (60), and outputs a second message if the current location lies within the second boundary surface (60), wherein: the first boundary surface (S60) surrounds the second boundary surface (60); at least the first and second boundary surfaces surround two markers; the first message differs from the second message; and the first boundary surface (S60) is distanced from the markers (1, 2, 3).

8. The marker detection device according to any one of claims 1 to 7, comprising a display apparatus (110) for displaying a location corresponding to the marker means on the basis of the calculated current relative location, even if the markers (1, 2, 3) are situated in one plane and if the current relative location is one of the second relative locations.

9. The marker detection device according to any one of the preceding claims, wherein outputting the message involves the warning apparatus (102) communicating an alarm signal and/or preventing the current relative location from being communicated.

## Revendications

1. Dispositif de navigation avec marqueurs, pour déterminer et/ou poursuivre la position d'un moyen de marquage, en particulier pour des applications médicales, en particulier pour la navigation ;
comprenant un moyen de marquage (20) qui comprend au moins deux marqueurs (1, 2, 3) avec des dimensions connues et dans une position relative connue l'un par rapport à l'autre ;
comprenant un moyen de détection (30, 30', 30") qui détecte le des signaux provenant desdits au moins deux marqueurs (1, 2, 3) ;
dans lequel le moyen de détection (30, 30', 30") est réalisé de telle façon que
dans des premières positions relatives (40,50, 60, S60) du moyen de détection (30', 30") par rapport au moyen de marquage (20) une détection de l'un des marqueurs (2) à l'aide du moyen de détection (30', 30") est entravée voire impossible en raison de la position d'un autre marqueur (1), et
dans les autres positions relatives, dites secondes positions relatives, par rapport au moyen de marquage (20), la totalité des marqueurs (1, 2, 3) du moyen de marquage (20) peuvent être détectés à l'aide du moyen de détection ;
comprenant un moyen de traitement de données (100),
**caractérisé en ce que** le moyen de traitement de données est réalisé de telle façon que
il calcule les premières positions relatives (40, 50, 60, S60) en se basant sur les positions relatives entre les marqueurs individuels (1, 2, 3) du moyen de marquage (20) et en se basant sur les dimensions des marqueurs (1, 2, 3) du moyen de marquage (20) ; et
en se basant sur les signaux détectés, sur les positions relatives connues des marqueurs (1, 2, 3) les uns par rapport aux autres, et sur les dimensions connues des marqueurs (1, 2, 3), il calcule la position relative actuelle entre le moyen de marquage (20) et le moyen de détection (30, 30', 30") et il détermine si la position relative actuelle est une position parmi les premières positions relatives (40, 50, 60, S60) ; et que
il est doté d'un moyen d'avertissement (102) qui émet une information quand la position relative actuelle est l'une des premières positions relatives (40, 50, 60, S60).

2. Dispositif de détection avec marqueurs selon la revendication 1, dans lequel les premières positions relatives (40, 50, 60, S60) satisfont l'une au moins des conditions suivantes :
a) elles se trouvent sur des droites qui traversent au moins deux des marqueurs ;
b) elles se trouvent sur des droites qui traversent l'un des marqueurs et qui passent à côté d'un autre des marqueurs à une distance prédéterminée ou à une distance inférieure à la distance prédéterminée ; et/ou
c) elles se trouvent sur des droites qui passent à côté de deux des marqueurs à une distance prédéterminée ou à une distance inférieure à la distance prédéterminée.

3. Dispositif de détection avec marqueurs selon la revendication 1 ou 2, dans lequel les premières positions relatives (40, 50, 60, S60) se trouvent sur des droites qui se recoupent entre deux marqueurs.

4. Dispositif de détection avec marqueurs selon l'une des revendications 1 à 3, dans lequel les premières positions relatives se trouvent à l'intérieur d'une enveloppe imaginaire (40, 50, 60, S60), ladite enveloppe (40, 50, 60, S60) entourant deux des marqueurs (1, 2, 3) et touchant l'un au moins des deux marqueurs (1, 2, 3), ou passant à une distance prédéterminée de l'un au moins des deux marqueurs (1, 2, 3).

5. Dispositif de détection avec marqueurs selon la revendication 4, dans lequel l'enveloppe entre les deux marqueurs présente une section transversale à surface minimum ou une section transversale ponctuelle (42, 52, 62) et s'élargit constamment en partant de celle-ci.

6. Dispositif de détection avec marqueurs selon la revendication 4 ou 5, dans lequel les marqueurs présentent une section ronde, ou l'enveloppe est de forme conique.

7. Dispositif de détection avec marqueurs selon l'une des revendications 1 à 6, dans lequel le moyen d'avertissement (102) émet une première information quand la position actuelle est l'une des premières positions relatives et se trouve à l'intérieur d'une première enveloppe imaginaire (S60) mais non pas à l'intérieur d'une seconde enveloppe imaginaire (60), et émet une seconde information quand la position actuelle se trouve à l'intérieur de la seconde enveloppe (60), ladite première enveloppe (S60) entoure la seconde enveloppe (60), au moins la première et la seconde enveloppe entourent deux marqueurs, la première information diffère de la seconde information, et la seconde enveloppe (S60) est à distance des marqueurs (1,2,3).

8. Dispositif de détection avec marqueurs selon l'une des revendications 1 à 7, comprenant un moyen d'affichage (110) pour afficher une position correspondant à l'un des moyens de marquage en se basant sur la position relative actuelle calculée, même si les marqueurs (1, 2, 3) se trouvent dans un plan et si la position relative actuelle est l'une des secondes positions relatives.

9. Dispositif de détection avec marqueurs selon l'une des revendications précédentes, dans lequel l'émission de l'information par le moyen d'avertissement (102) comprend la transmission d'un signal d'alarme et/ou l'interdiction de la transmission de la position relative actuelle.
